Europäisches Patentamt

European Patent Office

Office européen des brevets

(11) Publication number: **0 596 113 A1**

# EUROPEAN PATENT APPLICATION
## published in accordance with Art. 158(3) EPC

(21) Application number: 92902879.3

(22) Date of filing: **20.01.92**

(51) Int. Cl.⁵: **C12P 13/02**, C02F 3/34, //(C12P13/02,C12R1:645)

(86) International application number: **PCT/JP92/00038**

(87) International publication number: **WO 92/13087 (06.08.92 92/21)**

(30) Priority: **23.01.91 JP 82076/91**
**05.03.91 JP 123179/91**

(43) Date of publication of application:
**11.05.94 Bulletin 94/19**

(84) Designated Contracting States:
**DE IT**

(71) Applicant: **KABUSHIKI KAISHA KOBE SEIKO SHO**
**3-18 Wakinohama-cho 1-chome**
**Chuo-ku**
**Kobe 651(JP)**

(72) Inventor: **UESONO, Yukifumi**
**4-102, 39-ban,**
**Higashiikebukuro 4-chome**
**Toshima-ku Tokyo 107(JP)**
Inventor: **DEGUCHI, Tetsuya**

**7-302, 23-ban, Namiki 3-chome**
**Tsukuba-shi Ibaragi-ken 305(JP)**
Inventor: **NISHIDA, Tomoaki**
**1588-67, Sakuragaoka**
**Fujishiro-cho**
**Kitasoma-gun Ibaragi-ken 300-15(JP)**
Inventor: **TAKAHARA, Yoshimasa**
**29-8, Yatsu 5-chome**
**Narasino-shi Chiba-ken 275(JP)**
Inventor: **KATAYAMA, Yoshihiro**
**47-14, Inui 3-chome**
**Nerima-ku Tokyo 176(JP)**

(74) Representative: **Bühling, Gerhard, Dipl.-Chem. et al**
**Patentanwaltsbüro**
**Tiedtke-Bühling-Kinne & Partner**
**Bavariaring 4**
**D-80336 München (DE)**

(54) **METHOD OF DECOMPOSING POLYAMIDE.**

(57) A method of efficiently decomposing polyamides which have been difficult to decompose, such as water-insoluble high-molecular nylon, by treating the polyamide with a wood rotting basidiomycete, particularly a white rot basidiomycete, under the culturing conditions of an added nitrogen concentration of at most 0.1 g/l and/or an added carbon concentration of at most 0.2 g/l.

EP 0 596 113 A1

Field of the Invention

The present invention relates to a method of decomposing polyamide type high polymer compounds and, more particularly, to a novel industrial method for greatly decomposing even water-insoluble high polymer nylons, etc.

Therefore, not only the present invention greatly contributes to the treatment of plastic wastes which has now been a serious social problem but also it is much expected to be able to clarify the mechanism of decomposing the wastes to result in designing biodegradable high polymer compounds.

Prior Arts

Hitherto, as a means of decomposing polyamide compounds, methods of using bacteria (Flavobacterium sp. K172) has been known (Agr. Biol. Chem., 39 (6), 1219-1223 (1975); Fermentation Engineering, 60 (5), 363-375 (1982)).

However, all the conventional methods are ones of treating water-soluble low molecular weight nylon 6 oligomers (having a molecular weight of up to about 2000), and these could not be used for decomposing water-insoluble high molecular weight nylons (having a molecular weight of about 10,000 or more).

Problems to be Solved by the Invention

The problem which is especially significant in the treatment of plastic wastes is not the treatment of water-soluble plastics but the treatment of water-insoluble high polymer substances, and the same shall apply to polyamide type high polymer compound wastes. However, as will be obvious from the above-mentioned explanation, the conventional methods could not decompose water-insoluble nylons.

Means for Solving the Problems

The present invention has been made for the purpose of preventing environmental pollution by plastics, in view of the current technical situation as mentioned above, and the object of it is to develop a method capable of efficiently decomposing even water-insoluble polyamide type high polymer compounds which could not be decomposed by the conventional methods.

In order to attain the above-mentioned object, the present inventors earnestly investigated and directed their attentions to biological treatments of water-insoluble polyamide type high polymer compounds, following the conventional methods, from the viewpoint of preventing any secondary environmental pollution. However, even after they turned their attentions to bacteria in accordance with the conventional methods and investigated various bacteria, they could not find out the intended bacteria.

Under the situation, they investigated widely other various microorganisms and have noted wood-rotting fungi which have no relation to decomposition of plastics such as nylons, for the first time. However, they could not still attain the intended object and have noted the necessity of drastic change of the ideas regarding the culture conditions, the treating conditions, etc.

Quite contrary to the technical common sense, they tried to cut down nitrogen sources and/or carbon sources, which are essential nutrient components indispensable for growth and treatment of microorganisms and therefore must be added to the culture medium but should not be reduced, and tried to treat powdery polyamide type high polymer compounds with wood-rotting fungi and, as a result, they have succeeded, for the first time, in decomposing water-insoluble polymers, which could not heretofore be decomposed and treated by the conventional methods, and thus have completed the present invention. Now, the present invention will be explained in detail hereunder. Polyamide type high polymer compound may often be referred to as "nylon" herein.

In carrying out the present invention, polyamide type high polymer compounds to be decomposed and treated must be treated with wood-rotting basidiomycetous fungi by using a medium having a lowered nitrogen content and/or a lowered carbon content.

In the present invention, wood-rotting basidiomycetous fungi including brown-rotting basidiomycetous fungi, such as Lentinus lepideus, can be used widely, but white-rotting basidiomycetous fungi, that is, lignin-degrading microorganisms are especially advantageously used.

As examples of such wood-rotting basidiomycetous fungi, there are mentioned various microorganisms belonging to the following genera: Coriolus (e.g., Coriolus versicolor IFO 7043), Phanerochaete (e.g., Phanerochaete chrysosporium ATCC 34541), Trametes (e.g., Trametes dickinsii IFO 6488), Polyporus (e.g., Polyporus mikadoi IFO 6517), Stereum (e.g., Stereum frustulosum IFO 4932), Ganoderma (e.g., Ganoderma

2

applanatum IFO 6499), Lenzites (e.g., Lenzites betulina IFO 8714), Fomes (e.g., Fomes fomentarius IFO 30371), Porodisculus (e.g., Porodisculus pendulus IFO 4967), Lentinus (e.g., Lentinus edodes IFO 31366, L. lepideus IFO 7043), Serpula (e.g., Serpula lacrymans IFO 8697), etc.

As wood-rotting basidiomycetous fungi usable in the present invention, there is still mentioned NK-1148 strain (FERM BP-1859) in addition to the above-mentioned microorganisms. This strain may highly decompose polyamide type high polymer compounds.

The mycological characteristics of NK-1148 strain are mentioned below.

(1) State of Growth in Various Media:

| Culture Medium | State of Growth |
|---|---|
| Malt Extract Agar Medium | + + + |
| Potato-Glucose Agar Medium | + + + |
| Czapek's Agar Medium | + |
| Sabourand's Agar Medium | + + |
| Synthesized Mucor Agar Medium | + + |
| YpSs Agar Medium | + + + |
| Glucose-Dried Yeast Agar Medium | + + + |

Note-1, Culture Medium pH : 5.0 (before autoclave sterilization)

Note-2, Culture Condition : 28°C x 7 days

Note-3, State of Growth:

Weak: +

Medium: + +

Abundant: + + +

(2) Physiological and Morphological Properties:

① pH range for growth (cultivation in potato-glucose agar medium, 28°C, 4 days):
Grows at a pH of about 3-9, and not grow at pH 2 and 10. The optimum pH range for the growth is approoximately from 4 to 6.

② Temperature range for growth (cultivation in potato-glucose agar medium, pH 5, 4 days):
Grows at a temperature of about 10-45°C, and not grow at 50°C. The optimum temperature range for the growth is approximately from 28 to 37°C.

③ Phenol oxidase reaction (cultivation at 28°C, 4 days):
Shows weak or negative.

④ Morphology of Colony (cultivation in potato-glucose agar medium, pH 5, 28°C, 4 days):
White and felt-like.

In the present invention, polyamide type high polymer compounds are treated and decomposed with one or more kinds of the above-mentioned wood-rotting basidiomycetous fungi, whereupon the nitrogen concentration and/or the carbon concentration in the medium for cultivation are/is specifically controlled to limited range(s).

Particularly, a polyamide type high polymer compound is put on and/or is blended with a medium having a limited nitrogen concentration and/or a limited carbon concentration, and inoculated with a wood-rotting basidiomycetous fungus and incubated statically at a suitable temperature of, for example, from 15 to 35°C, whereupon the high polymer compounds can be decomposed in about 5 to 30 days. Polyamide type high polymer compounds may also be decomposed even in the liquid cultivation under the above-mentioned conditions (with aerating and/or stirring).

In the case, though the microorganisms are cultivated, it is important that the nitrogen concentration and/or the carbon concentration in the medium are/is limited. The nitrogen concentration and/or the carbon concentration are/is the lowest possible one(s), and preferably, the medium for cultivating them does not contain carbon and nitrogen sources. From the industrial viewpoint, the nitrogen concentration may well be 0.1 g/liter or less, more desirably 0.05 g/liter or less, and the carbon concentration may well be 0.2 g/liter or less, for the purpose of attaining favorable results. The other components than nitrogen and carbon of constituting the medium are not specifically defined and the components which are generally employed for cultivating wood-rotting fungi can be used suitably. The present invention may attain the intended object either by solid cultivation or by liquid cultivation, provided that the above-mentioned conditions relating to nitrogen and/or carbon are satisfied. Therefore, even when polyamide type high polymer compounds and wood-rotting fungi are added to and incubated in a medium completely free from nitrogen sources and carbon sources, for example, only water (to which, if necessary, agar and/or a pH adjusting agent or the like may be added), the polyamide type high polymer compounds can be decomposed.

In the present invention, wood-rotting basidiomycetous fungi are used. Not only mycelia of such fungi but also cultures of them and/or processed products of them may be used. The culture widely means a mixture comprising the mycelia of cultivated fungus and the broth. In the present invention, the mycelia in the form of, for example, a wet cake as separated from the culture, the resulting residue, and the broth from which all solids have been removed may be used. The processed products mean all the concentrated products, the dried products and the diluted products of the above-mentioned materials.

In decomposing and treating polyamide type high polymer compounds in accordance with the present invention, they may directly be treated as they are, but they are preferably pulverized, powdered or filmed into films with fine pores before the treatment, for the purpose of increasing the contact area of them with the microorganisms or with the enzymes to be produced from them. Coating of the polyamide type high polymer compounds with surfactants or blending of them for the purpose of improving the wettability of the high polymer compounds is more preferred.

In accordance with the present invention, all water-insoluble polyamide type high polymer compounds (for example, those having a molecular weight of approximately from 80,000 to 160,000) including linear-chain synthetic polyamides of nylon mm, nylon mn and nylon n and other polyamides can be decomposed. Therefore, by the present invention, nylon 6, nylon 66, nylon 610, nylon 7, nylon 9, nylon 11, nylon 12 and the like may well be decomposed. As a matter of course, mixture of them may be decomposed by biological treatment. Next, examples of the present invention will be mentioned below.

EXAMPLE 1

A nylon membrane with fine pores (Nylon 66 Membrane Filter, produced by Sartorius Co.) was put on a nitrogen source-free solid medium ($KH_2PO_4$ 1.0 g; $NaH_2PO_4$ 0.2 g; $MgSO_4.7H_2O$ 0.1 g; $ZnSO_4.7H_2O$ 0.01 mg; $CuSO_4.5H_2O$ 0.02 mg; dimethyl succinate 1.46 g; glucose 20 g; agar 30 g; water 1 liter), and incubated with various microorganisms (white-rotting basidiomycetous fungi : Phanerochaete chysosporium ATCC 34541, Coriolus versicolor IFO 7043, NK-1148 FERM BP-1859, brown-rotting basidiomycetous fungi: Lentinus lepideus IFO 7043, Serpula lacrymans EPRI 6352, imperfect fungi: Aspergillus niger IFO 6341, Penicillium citrinum IFO 6352, bacteria : Bacillus subtilis IFO 3034, Pseudomonas paucimobillis SYK-6) and incubated statically at 20 to 28°C for 20 days. After incubation, the treated porous membrane was dissolved in HFIP (hexafluoroisopropanol) to be a solution having a sample concentration of 0.2 % and the resulting solution was analyzed by GPC (column of HFIP-80M Model, manufactured by Showa Denko KK; eluent of HFIP; flow rate of 1 ml/min; temperature of 40°C; monitor of RI) to evaluate the decomposition, if any, of the membrane by determining the variation of the average molecular weights of the treated sample. Table 1 below shows average molecular weights of the sample as treated with the microorganisms.

Table 1 Nylon Decomposing Capacity of Various Microorganisms

| Microorganism Used | Weight Average Molecular Weight | Number Average Molecular Weight |
|---|---|---|
| Control (not treated with microorganism) | 84,800 | 49,700 |
| Wood-rotting basidiomycetous fungi | | |
| White-rotting basidiomycetous fungi | | |
| P. chrysosporium | 11,500 | 4,900 |
| C. versicolor | 27,100 | 8,200 |
| NK-1148 | 5,500 | 2,300 |
| Brown-rotting basidiomycetous fungi | | |
| L. lepideus | 62,000 | 29,100 |
| S. lacrymans | 84,800 | 49,700 |
| Imperfect fungi | | |
| Aspergillus niger | 84,800 | 42,200 |
| Penicillium citrinum | 82,100 | 32,800 |
| Bacteria | | |
| Bacillus subtilis | 84,800 | 49,700 |
| Ps. paucimobillis | 84,800 | 49,700 |

EXAMPLE 2

A nylon membrane was inoculated with each of three different white-rotting basidiomycetous fungi (Phanerochaete chysosporium, Coriolus versicolor, NK-1148), which were identified to decompose the nylon sample in Example 1, and incubated statically in the same manner as in Example 1, except that the nitrogen concentration in the solid medium was varied. Particularly, the nitrogn concentration in the medium was varied to be 0 g/liter, 0.05 g/liter, 0.10 g/liter and 0.15 g/liter with ammonium tartrate, and the other components in the medium were same as those in Example 1. After incubation, the decomposition of the sample, if any, by the fungi was determined by GPC. The results obtained are shown in Table 2 below.

Table 2    Influence of Nitrogen Concentration in Medium on Decomposition of Nylon

| Nitrogen concentration | P. chrysosporium | | C. versicolor | | NK-1148 | |
|---|---|---|---|---|---|---|
| | Weight Average Molecular Weight | Number Average Molecular Weight | Weight Average Molecular Weight | Number Average Molecular Weight | Weight Average Molecular Weight | Number Average Molecular Weight |
| 0.15 g/liter | 84,800 | 49,700 | 81,400 | 41,700 | 83,300 | 40,800 |
| 0.10 g/liter | 68,600 | 29,800 | 80,300 | 41,200 | 68,800 | 30,100 |
| 0.05 g/liter | 44,200 | 24,200 | 57,900 | 44,900 | 45,100 | 28,000 |
| 0.0 g/liter | 11,500 | 4,900 | 27,100 | 8,200 | 5,500 | 2,300 |

Note : Control (not treated with microorganism)

Weight Average Molecular Weight : 84,800

Number Average Molecular Weight : 49,700

## EXAMPLE 3

A nylon membrane was inoculated with the white-rotting basidiomycetous fungus (NK-1148), which was identified to decompose the nylon sample in Example 1, and incubated statically in the same manner as in Example 1, except that the carbon concentration in the solid medium was varied. Particularly, the carbon concentration in the medium was varied to be 0 g/liter, 0.2 g/liter, 0.4 g/liter and 8.0 g/liter with glucose as added to the medium, and dimethyl succinate and $(NH_4)_2SO_4$ in the medium were used in an amount of 0 g and 0.58 g, respectively. The other components in the medium were same as those in Example 1. After incubation, the decomposition of the sample, if any, by this fungus was determined by GPC. The results obtained are shown in Table 3 below.

Table 3

| Influence of Carbon Concentration in Medium on Decomposition of Nylon | | |
|---|---|---|
| Carbon Concentration | Weight Average Molecular Weight | Number Average Molecular Weight |
| 8.0 g/liter | 83,300 | 40,800 |
| 0.4 g/liter | 81,200 | 36,200 |
| 0.2 g/liter | 11,000 | 4,300 |
| 0.0 g/liter | 6,000 | 2,500 |
| Note : <br> Control (not treated with microorganism) <br> Weight Average Molecular Weight: 84,800 <br> Number Average Molecular Weight: 49,700 | | |

## EXAMPLE 4

A nylon membrane was inoculated with the white-rotting fungus (NK-1148), which was identified to decompose the nylon sample in Example 1, and incubated statically in the same manner as in Example 3, except that a solid medium containing neither nitrogen sources nor carbon sources was used. Precisely, the amount of glucose and that of $(NH_4)_2SO_4$ in the medium were both 0 (zero) g, and the other components in the medium were same as those in Example 3. After incubation, the decomposition of the sample, if any, was determined by GPC. In addition, a nylon membrane was also incubated statically in a solid medium (agar 30 g; water 1 liter) as prepared by removing all the nutrient sources from the medium of Example 3, whereupon the decomposition of the sample, if any, was also determined in the same manner by GPC. The results obtained are shown in Table 4 below.

Table 4

| Influence of Nitrogen Concentration and Carbon Concentration in Medium on Decomposition of Sample | | | |
|---|---|---|---|
| Carbon Concentration (g/liter) | Nitrogen Concentration (g/liter) | Weight Average Molecular Weight | Number Average Molecular Weight |
| 0.0 | 0.0 | 5,000 | 2,200 |
| 0.0 | 0.0 (All nutrient sources were removed.) | 5,500 | 2,300 |
| 0.0 | 0.15 | 6,000 | 2,500 |
| 8.0 | 0.0 | 5,500 | 2,300 |
| 8.0 | 0.15 | 83,300 | 40,800 |
| Note : <br> Control (not treated with microorganism) <br> Weight Average Molecular Weight: 84,800 <br> Number Average Molecular Weight: 49,700 | | | |

7

EXAMPLE 5

Nylon samples were inoculated with Phanerochaete chysosporium, and incuabated under the nitrogen condition showing the greatest decomposing capacity in Example 2 (the medium used contained 0 g/liter of ammonium tartrate and the other conditions were same as those in Example 1), whereupon nylon samples of various forms were decomposed with the fungus to determine the influence of the shapes of the nylon samples on the decomposition of them. Particularly, four kinds of nylon samples of a porous nylon membrane (Nylon 66 Membrane Filter, produced by Sartorius Co.), a nylon film (produced by Asahi Chemical Industry Co.; thickness of 50 $\mu$m), nylon 66 fibers (produced by NBC Industry Co.; diameter of 60 $\mu$m) and a powder sample were used to be decomposed with the fungus. As the powder sample, a nylon sample was powdered with a sample mill (manufactured by HEIKO CO.; TI-100 Model) repeatedly 4 to 5 times each for 5 minutes, and the resulting powder was classified. Thus, a 80 mesh-pass fraction from the classification was used as the powder sample to be decomposed. The decomposition, if any, of the tested samples was evaluated in the same manner as in Example 1. The results obtained are shown in Table 5 below.

Table 5    Influence of Shapes of Nylon Samples on Decomposition of Them

| | | Not treated with microorganism | Treated with microorganism |
|---|---|---|---|
| Powder (80 mesh-pass) | Weight Average Molecular Weight | 60,000 | 26,300 |
| | Number Average Molecular Weight | 17,200 | 9,400 |
| Porous Membrane | Weight Average Molecular Weight | 84,800 | 11,500 |
| | Number Average Molecular Weight | 49,700 | 4,900 |
| Nylon Film | Weight Average Molecular Weight | 61,000 | 57,300 |
| | Number Average Molecular Weight | 19,200 | 17,700 |
| Nylon Fibers | Weight Average Molecular Weight | 64,100 | 61,400 |
| | Number Average Molecular Weight | 8,600 | 8,600 |

Advantage of the Invention

In accordance with the present invention, polyamide type high polymer compounds such as water-insoluble high molecular weight polymers may efficiently be decomposed without causing any secondary

9

environmental pollution. Therefore, the present invention greatly contributes to the treatment of plastic wastes which is now a worldwide serious social problem.

Reference to Microorganism as Deposited under Rule 13.2:

1. NK-1148
   a. Name and address of the organization in which the microorganism was deposited.
      Name:         Fermentation Research Institute, Agency of Industrial Science and Technology, Ministry of International Trade and Industry
      Address:      1-3, Higashi I-chome, Tsukuba-shi, Ibaraki-ken 305, Japan
   b. Date of deposition of the microorganism in the organization of a.
      May 23, 1987
   c. Deposit number of the microorganism as rendered by the organization of a.
      FERM BP-1859

**Claims**

1. A method of decomposing a polyamide type high polymer compound, in which the polyamide type high polymer compound is treated with a wood-rotting basidiomycetous fungus under the culture condition of a medium having an added nitrogen concentration of 0.1 g/liter or less and/or an added carbon concentration of 0.2 g/liter or less.

2. The method of decomposing a polyamide type high polymer compound as claimed in claim 1, in which the wood-rotting basidiomycetous fungus is a white-rotting basidiomycetous fungus.

3. The method as claimed in claim 1 or 2, in which the polyamide type high polymer compound to be decomposed is one as pulverized, powdered and/or filmed into porous membrane.

# INTERNATIONAL SEARCH REPORT

International Application No  PCT/JP92/00038

**I. CLASSIFICATION OF SUBJECT MATTER** (if several classification symbols apply, indicate all) [6]

According to International Patent Classification (IPC) or to both National Classification and IPC

Int. Cl[5]  C12P13/02, C02F3/34
         (C12P13/02, C12R1:645)

**II. FIELDS SEARCHED**

| Minimum Documentation Searched [7] | |
|---|---|
| Classification System | Classification Symbols |
| IPC | C12P13/02, C02F3/34 |

| Documentation Searched other than Minimum Documentation to the Extent that such Documents are Included in the Fields Searched [8] |
|---|
| Biological Abstracts Data Base (BIOSIS) |

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | Agricultural and Biological Chemistry, Volume 39, no.6, June 6, 1975 (06. 06. 75), H. OKada et al.: "Utilization of a Cyclic Dimer and Linear Oligomers of e-Aminocaproic Acid by Achromobacter guttatus KI72", see p. 1219 - 1223 | 1-3 |

* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search | Date of Mailing of this International Search Report |
|---|---|
| April 03, 1992 (03. 04. 92) | April 28, 1992 (28. 04. 92) |
| International Searching Authority | Signature of Authorized Officer |
| Japanese Patent Office | |

Form PCT/ISA/210 (second sheet) (January 1985)